# EUROPÄISCHE PATENTSCHRIFT

(11) **EP 1 784 140 B1**
(45) Veröffentlichungstag und Bekanntmachung des Hinweises auf die Patenterteilung: **01.02.2012**
(21) Anmeldenummer: 05763680.5
(22) Anmeldetag: 21.07.2005
(51) Int. Cl.: A61F 2/00

(54) **VORRICHTUNG ZUM VERSCHLIESSEN EINES NATÜRLICHEN ODER KÜNSTLICHEN DARMAUSGANGS**
DEVICE FOR CLOSING A NATURAL OR ARTIFICIAL ANUS
DISPOSITIF POUR FERMER UN ANUS NATUREL OU ARTIFICIEL

(30) Priorität: 23.07.2004 WO PCT/EP2004/008256; 09.02.2005 DE 102005005988; 15.04.2005 DE 102005017652; 06.05.2005 DE 102005021081
(43) Veröffentlichungstag der Anmeldung: 16.05.2007
(73) Patentinhaber: Advanced Medical Balloons Gmbh, 60313 Frankfurt am Main (DE)
(72) Erfinder: GÖBEL, Fred, 69259 Wilhelmsfeld (DE)
(74) Vertreter: Küchler, Stefan
(86) Internationale Anmeldenummer: PCT/EP2005/007950
(87) Internationale Veröffentlichungsnummer: WO 2006/010556

(56) Entgegenhaltungen:
- US-A- 3 802 418
- US-A- 4 241 735
- US-A- 4 344 434
- US-A- 4 555 242
- US-A- 4 686 985
- US-A1- 2004 044 307
- US-B1- 6 527 755

## Beschreibung

### Technisches Gebiet

Die Erfindung betrifft eine Vorrichtung zum Verschließen eines natürlichen oder künstlichen Darmausgangs, umfassend einen aufblasbaren Ballon mit einer etwa torusförmigen Struktur, gebildet aus einem flächigen, in sich umgestülpten Schlauchabschnitt, dessen beide Enden etwa koaxial ineinander verlaufen und mit (je) einer Hülse verbunden sind, wobei die äußere Lage des umgestülpten Schlauchabschnittes einen radial erweiterten, patienten proximalen Bereich zum Einführen in das Rektum und einen demgegenüber verjüngten, patientendistalen Bereich aufweist, der während des Gebrauchs außerhalb des Rektums verbleibt, und wobei die Schlauchabschnitte in dem transanalen Bereich eine nach Shore-Härteprufüng A bestimmbare Materialhärte H₁ aufweisen, die größer ist als 60.

### Stand der Technik

Solche, in sich umgestülpte und sodann durch Aufblasen zu einem Ballon entfaltete Schlauchabschnitte können mit Vorteil zum Verschließen des Rektums oder eines Anus Praeter verwendet werden. Beim Aufblasen des Ballons wird jedoch die innere Lage des Schlauchabschnittes nach innen gepreßt, so dass ein dortiges Lumen verschlossen ist. Andererseits besteht ein Bedürfnis danach, den Darm in regelmäßigen Zeitabständen unter minimalem Aufwand zu entleeren, d.h., möglichst ohne Entfernen des Darmverschlusses und anschließendem Wiedereinsetzen desselben, was aber aufgrund der inneren, verschlossenen Schlauchlage nicht ohne weiteres möglich ist.

In der US 4,555,242 A ist eine Vorrichtung zum Abfließenlassen körperlicher Abfallstoffe aus einer natürlichen oder künstlichen Blase in einem Patientenkörper durch eine Öffnung, die mit der Blase durch einen Kanal verbunden ist, beschrieben. Die Vorrichtung enthält eine Leitungsröhre, welche durch die Öffnung und den Kanal in die Blase eingesetzt werden kann. Dadurch wird ermöglicht, dass körperliche Abfallstoffe aus der Blase durch die Öffnung aus dem Körper herausgeführt werden können. Die Leitungsröhre weist ein distales Ende auf, welches in der Blase aufgenommen werden kann, sowie ein proximales Ende, welches axial von dem distalen Ende beabstandet ist, so dass sich das proximale Ende durch die Öffnung zur Körperaußenseite erstreckt. Des Weiteren weist die Vorrichtung externe Rückhaltemittel auf, die an dem proximalen Ende der Leitungsröhre angeordnet sind, um die Öffnung um das proximale Ende herum abdichten zu können und einen Auslass für Abfallstoffe zu bilden, welche aus dem proximalen Ende abfließen. Außerdem enthält die Vorrichtung einen aufblasbaren Ballon, welcher das Äußere der Leitungsröhre entlang eines wesentlichen Teils ihrer Länge vom distalen Ende her einschließt, wobei der Ballon einerseits einen wulstartigen Bereich an der Spitze aufweist, welcher das distale Ende umgibt, sowie andererseits einen gerippten Bereich, der sich proximal von dem Bereich an der Spitze erstreckt. Dabei bilden der Ballon und die Leitungsröhre einen Raum zwischen sich aus. Schließlich enthält die Vorrichtung auch noch einen Durchlass, welcher mit dem Raum in Verbindung steht. Dadurch wird ein Durchflussweg in den Raum für ein Fluid geschaffen, so dass der Ballon aufgeblasen werden kann. Die Wand des Ballons ist in dem Bereich der Spitze dicker als in dem gerippten Bereich, so dass der gerippte Bereich im wesentlichen vollständig aufgeblasen werden kann, bevor der Bereich an der Spitze aufgeblasen wird, wenn sich der gerippte Bereich in dem Kanal und der Bereich an der Spitze in der Blase befinden.

### Darstellung der Erfindung

Der Erfindung liegt die Aufgabe zugrunde, eine gattungsgemäße Vorrichtung zum Verschließen eines natürlichen oder künstlichen Darmausgangs derart weiterzubilden, dass eine kontinuierliche Entleerung des Darms ohne Entfernen des Darmverschlusses möglich ist.

Diese Aufgabe löst die Erfindung durch die vorrichtung gemäß der unabhängigen Patentanspruchs 1.

Die Erfindung sicht vor, dass innerhalb des patientenproximalen Bereichs der äußeren Lage eine Versteifungshülse derart eingesetzt ist, dass diese von dem Hohlraum innerhalb des Ballons durch dessen innere, durchgängige Lage vollständig getrennt ist, wobei die Versteifungshülse mit keinem der Enden des den Ballon bildenden Schlauchabschnitts verbunden ist.

Diese Hülse hält daher das innere Lumen in dem patientenproximalen Bereich des Ballons ständig geöffnet und erleichtert daher die natürliche Darmentleerung. Der transanale Bereich kann dagegen nach Art eines Ventils geschlossen, aber auch geöffnet werden.

Es liegt im Rahmen der Erfindung, dass die nach Shore-Härteprüfung A bestimmbare Materialhärte H₁ der Schlauchabschnitte in dem transanalen Bereich größer ist als 70, vorzugsweise größer als 80, so dass einerseits eine Herstellung durch Extrudieren möglich ist und sich andererseits beim Aufblasen des Ballons eine vorhersagbare, in ihrem Ausmaß begrenzte Dehnung einstellt.

Die Wandstärke des Ballons sollte, insbesondere im Bereich seiner äußeren Lage, kleiner sein als 50 µm, vorzugsweise kleiner als 40 µm, insbesondere kleiner als 30 µm. Dadurch ergibt sich trotz der relativ hohen Materialhärte H₁ eine ausreichende Geschmeidigkeit, so dass dieser Bereich beim Einführen und zwischen den Entleerungphasen auf einen minimalen Querschnitt zusammengefaltet werden kann.

Die Erfindung empfiehlt, für einen den Ballon bildenden Schlauchabschnitt Polyurethan zu verwenden. Dieses Material hat einerseits die erforderliche Materialhärte und läßt sich entsprechend dem gewünschten Umfang präformieren; es läßt sich überdies mit einer sehr dünnen Wandstärke herstellen.

Es hat sich bewährt, dass die Versteifungshülse eine nach Shore-Härteprüfung A bestimmbare Materialhärte H₂ aufweist, die gleich oder kleiner ist als die nach Shore-Härteprüfung A bestimmbare Materialhärte H₁ der Schlauchabschnitte in dem transanalen Bereich. Da die Versteifungshülse bedeutend dicker ausgeführt ist als der Ballon, und andererseits mit demselben zusammen oftmals bis über 30 Tage hinaus im Rektum eines Patienten verbleiben soll, darf sie nicht zu hart sein, um Irritationen oder gar Verletzungen der Darmschleimhaut zu vermeiden. Dies wird durch eine begrenzte Härte H₂ des Hülsenmaterials erreicht.

Es bietet sich an, den Schlauch und die Versteifungshülse aus dem selben Material zu fertigen. Durch die Verarbeitung identischer Materialien läßt sich in der Regel der maschinelle Fertigungsaufwand deutlich reduzieren; andererseits kann eine Verbindung leicht und in der Regel zuverlässig durch vorübergehendes Anlösen der beiden miteinander zu verbindenden Teile mittels eines für das betreffende Material geeigneten Lösungsmittels bewirkt werden. Auf kostspielige Kleberzubereitungen kann somit verzichtet werden.

Es hat sich als günstig erwiesen, dass die Materialhärte H₂ und Wandstärke d der Versteifungshülse derart gewählt sind, dass diese zum Einführen in das Rektum radial zusammengedrückt werden kann. Diese beiden Parameter beeinflussen die tatsächliche Härte der Versteifungshülse, bspw. multiplikativ. Das bedeutet, je härter das Material der Versteifungshülse ist, um so dünnwandiger muß diese ausgebildet sein, und umgekehrt.

Die Versteifungshülse sollte mit keinem Ende des den Ballon bildenden Schlauchabschnitts verbunden sein, so dass sie jeder Bewegung des vorderen, patientenproximalen Ballonteils folgen kann. Dadurch behält dieser Ballonabschnitt seine hochgradige Bewegungsfreiheit und kann - infolge des inneren Druckes beim Aufblasen - nach außen und/oder rückwärts rollen, was in diesem Fall erwünscht ist, wie weiter unten noch ausgeführt wird.

Dieser Erfindungsgedanke läßt sich dahingehend weiterbilden, dass ein oder vorzugsweise beide Enden des den Ballon bildenden Schlauchabschnitts in dem oder jenseits (patientenfem) des transanalen Bereich(s) liegen. Die beiden Enden des Schlauchabschnittes liegen somit außerhalb des Rektums, so dass diese Bereiche gut zugänglich sind und sich überdies keine Dichtungsprobleme ergeben.

Vorzugsweise ist eine mit wenigstens einem Ende des den Ballon bildenden Schlauchabschnitts verbundene Hülse als extrakorporales Konnektorstück ausgebildet. Daran können sodann verschiedene Instrumente angeschlossen werden, bspw. Spülvorrichtungen, in einen patientenfemen Auffangbehälter abführende Katheter, etc.

Indem das abgeschlossene Volumen innerhalb des Ballons von außen mit Druck beaufschlagbar ist, läßt sich dessen geometrische Form gezielt beeinflussen.

Efindungsgemäß wird ein von dem Ballon umgebener Durchgang zur Entleerung des Darms dadurch geschaffen, dass das mit Druck beaufschlagbare Volumen durch zwei etwa konzentrisch zueinander liegende Oberflächenbereiche des Ballons begrenzt wird. Daher kann auf einen Schaft od. dgl. völlig verzichtet werden; dessen Funktion wird vollständig von den konzentrischen Lagen des Ballons übernommen.

In Weiterverfolgung dieses Erfindungsgedankens kann weiter vorgesehen sein, dass zur Offenhaltung des Enbeerungs-Durchgangs in demjenigen Bereich der Vorrichtung, die in dem Analkanal liegt, der innere Oberflächenbereich des Ballons punkt-, linien- oder flächenmäßig mit dem äußeren Oberflächenbereich des Ballons verbunden ist, bspw. durch Verschweißen oder Verkleben. Solchenfalls wird die innere Ballon- bzw. Schlauchlage an der äußeren Lage fixiert.

Die Erfindung zeichnet sich weiterhin aus durch einen Okklusionsballon zum Verschließen des Entleerungs-Durchgangs. Ein derartiger Okklusionsballon ist im Bereich des zentralen Lumens des eigentlichen Ballons fixiert und kann getrennt von dem eigentlichen Ballon entfaltet werden, bspw. über eine getrennte Zuleitung, Ober die ein vorzugsweise gasförmiges Medium in den Okklusionsballon geleitet wird.

Bevorzugt ist der Okklusionsballon innerhalb der Versteifungshülse positioniert, wo er in deflatiertem Zustand den Durchgang freigibt.

Die Erfindung sieht weiterhin vor, dass der Okklusionsballon aus einem dünnwandigen, entsprechend präformierten Material gebildet ist. Damit läßt sich die Form des Okklusionsballons in dessen inflatiertem Zustand ziemlich exakt vorgeben, und der zum Entfalten des Ballons erforderliche Druck kann relativ niedrig gehalten werden, da er keine elastische Dehnung des Ballonmaterials bewirken muß.

Im Rahmen einer alternativen Ausführungsform der Erfindung kann der Okklusionsballon aus einem völlig elastisch zurückstellbaren, zum Ballon ausformbaren Kompartiment gebildet sein. Dies ist möglich, weil der Okklusionsballon innerhalb des zentralen Lumens der erfindungsgemäßen Vorrichtung positioniert ist und daher nicht in Kontakt mit menschlichem Gewebe gelangt, so dass selbst bei einem zu hohen Innendruck keine Schädigungen an menschlichem Gewebe verursacht werden könnten.

Mit großem Vorteil ist im vorderen Bereich des Ballons, insbesondere im Bereich der Versteifungshülse oder gar patientenproximal derselben, eine Spülöffnung vorgesehen, die mit einem entlang des transanalen Abschnitts verlaufenden Kanal zum Einleiten eines Spülfluids verbunden ist. Damit ist es möglich, ohne Entfernung der erfindungsgemäßen Vorrichtung jederzeit einen Darmeinlauf machen zu können.

Wenn sich der Kanal zum Einleiten eines Spülfluids bis zu der vorderen, patientenproximalen Ballonschulter erstreckt, so werden bei einem Einlauf keinerlei Bakterien aus dem transanalen Bereich in den Darm gespült, sondern das Spülfluid gelangt ohne jegliche Verunreinigung direkt in den Darm.

Die Erfindung erfährt eine weitere Optimierung durch eine radiale Erweiterung in dem transanalen Abschnitt. Diese Erweiterung soll auch als Gegenstück zu der ballonförmigen Erweiterung in dem intrarektalen Schlauchabschnitt dienen. Ihre Aufgabe besteht darin, bei Auftreten einer durch das Aufblasen des Ballons entstehenden Zugkraft den transanalen Bereich der Vorrichtung zumindest teilweise außerhalb des Anus zu halten, so dass sich eine definierte Lage der erfindungsgemäßen Vorrichtung ergibt.

Zu diesem Zweck kann die äußere Lage des den Ballon bildenden Schlauchabschnitts in dem transanalen Bereich eine Präformierung mit einer nach außen weisenden Erweiterung aufweisen. Demzufolge bildet ein entsprechend präformierter Abschnitt des Ballons selbst das transanale bzw. präanale Widerlager für die von der intrarektalen, ballonförmigen Erweiterung erzeugte, zum Anus hin wirkende Axialkraft.

Bei einer vorteilhaften Weiterbildung der Erfindung weist die nach außen gerichtete Präformierung in dem transanalen Bereich der äußeren Lage des Ballons eine ring- oder scheibenförmige Geometrie auf. Dadurch stellen sich unabhängig von der Verdrehposition der erfindungsgemäßen Vorrichtung stets optimale Verhältnisse ein.

Andererseits ist es auch möglich, dass die nach außen weisende Präformierung in dem transanalen Bereich der äußeren Lage des Ballons die Geometrie eines oder zweier, etwa diametral voneinander weg divergierender Finger aufweist. Diese fingerartigen Fortsätze lassen sich in der Analfalte plazieren, so dass der Tragekomfort maximal ist.

Weiterhin kann über der äußeren Lage des den Ballon bildenden Schlauchabschnitts in dem transanalen Bereich bzw. im Bereich des Übergangs vom transanalen Bereich zum Konnektorstück ein keilförmiges Element vorgesehen, insbesondere befestigt sein. Auch ein derartiges Schaumstoff-Element kann als Wideriager dienen und weist zudem eine erhöhte Steifigkeit auf mit einer daraus resultierenden, sehr guten Lagestabilität.

### Kurzbeschreibung der Zeichnung

Anhand einiger Ausführungsbeispiele wird die Erfindung nachstehend näher erläutert.

Es zeigen:
- Figur 1: eine erste Ausführungsform der Erfindung in einer perspektivischen Darstellung;
- Figur 2: einen Längsschnitt durch die Fig. 1 entlang der Linie II - II; und
- Figur 3: eine der Fig. 2 entsprechende Darstellung einer abgewandelten Ausführungsform der Erfindung, teilweise abgebrochen.

### Ausführung der Erfindung

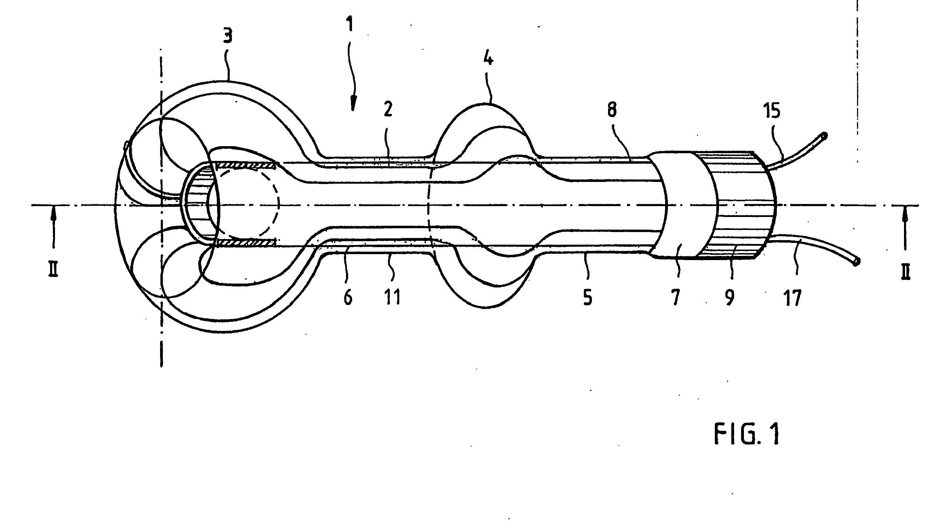

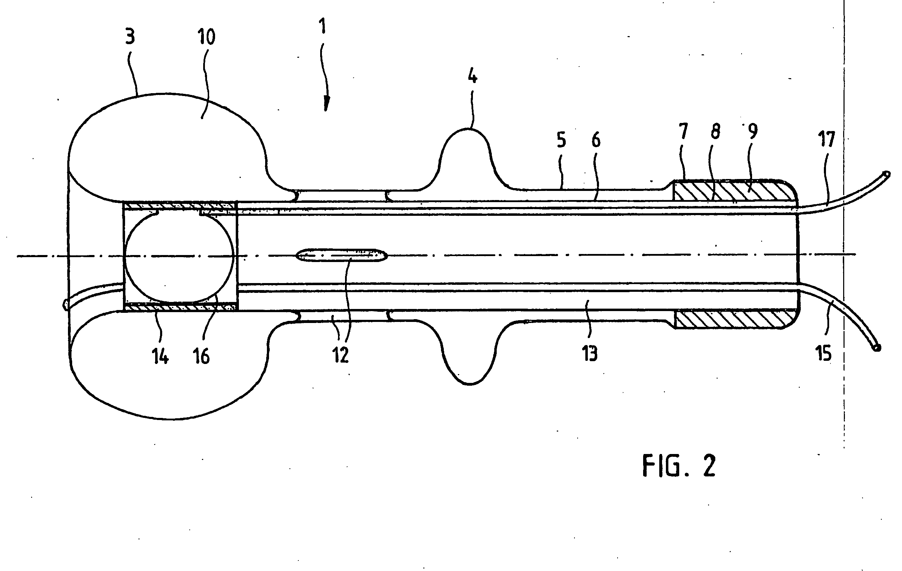

Die erfindungsgemäße Vorrichtung 1 nach Fig. 1 und 2 dient zum Verschließen eines natürlichen oder künstlichen Darmausgangs. Sie umfaßt einen präformierten und in sich zurückgestülpten Schlauch 2 aus einem dünnwandigen Material, bspw. aus Polyurethan mit einem Shore-Härtegrad A 90 und einer Wandstärke von weniger als 25 µm.

Durch die Präformierung hat der Schlauch 2, dessen ursprünglicher Durchmesser etwa zwischen 15 mm und 30 mm liegt, zwei radiale Erweiterungen 3, 4 erhalten. Die eine, vorzugsweise größere Erweiterung 3 befindet sich etwa in der Mitte des Schlauchs, die nach der Umstülpung das patientenproximale Ende der Vorrichtung 1 bildet. Die andere Präformierung 4 befindet sich etwa in der Mitte des nach dem Umstülpen außen liegenden Schlauchabschnitts 5, während der innen liegende Schlauchabschnitt 6 keine Erweiterung aufweist, sondern einen gleichbleibenden Querschnitt hat.

Die beiden freien Enden 7, 8 der beiden Schlauchabschnitte 5, 6 sind mit einem hülsenförmigen Konnektorstück 9 verbunden. Das Konnektorstück 9 kann - insbesondere im Bereich seines patientendistalen Endes - ein Innen- und/oder Außengewinde aufweisen zum Anschluß verschiedener medizinischer Apparaturen, bspw eines Entleerungsbeutels, eines Katheters od. dgl.

Vorzugsweise ist die Außenlage 5 des Ballons 2 an der Außenseite des Konnektorstücks 9 festgeklebt, die Innenlage 6 des Ballons 2 an dessen Innenseite. Dadurch ist der Hohlraum 10 zwischen der Innen- und Außenlage 5, 6 des Ballons 2 luftdicht abgeschlossen; lediglich im Bereich des Konnektorstücks 9 befindet sich eine in der Zeichnung nicht dargestellte Verbindung nach außen, an welche eine Quelle mit einem mit Druck beaufschlagbaren Medium anschließbar ist, um den Ballon 2 zu entfalten.

Durch die vergleichsweise große Materialhärte des Ballons 2 ist dieser nur geringfügig elastisch und nimmt in inflatiertem Zustand seine in Fig. 1 erkennbare, durch die Präformierung vorgegebene Gestalt an. Diese umfaßt eine etwa zylindrische Grundgestalt mit einer etwa kugelförmigen Erweiterung 3 an dem patientenproximalen, dem Konnektorstück 9 gegenüberliegenden Ende und mit einer ring- oder scheibenförmigen Erweiterung 4 etwa in der Mitte zwischen den beiden Enden 3, 9.

Die kugelförmige Erweiterung 3 wird in deflatiertem Zustand in dem Rektum eines Patienten plaziert (intrarektaler Abschnitt), während der sich anschließende, zylindrische Abschnitt 11 bis zu der ring- oder scheibenförmigen Erweiterung 4 durch den Analkanal nach außen führt (transanaler Bereich), die patientendistale Erweiterung 4 liegt unmittelbar vor dem Anus präanal in der Analfalte.

In dem transanalen Bereich 11 zwischen den beiden Erweiterungen 3, 4 sind die beiden Lagen des Ballons 2, nämlich die Außenlage 5 und die Innenlage 6 miteinander verbunden, vorzugsweise durch Verschweißungen 12 oder Verklebungen. Es kann sich hierbei um punkt-, linien- oder flächenförmige Verschweißungen handeln. Bei der dargestellten Ausführungsform sind vier in axialer Richtung verlaufende Schweißlinien 12 vorgesehen, die jeweils um etwa gleiche Umfangswinkel gegeneinander versetzt sind. Dank dieser Schweißverbindungen 12 kann das innere Lumen 13 innerhalb der Innenlage 6 des Ballons 2 leichter geöffnet werden, wenn der Darm entleert werden soll.

Kommt es bei der spontanen Entleerung des Enddarms zu einer Kontraktionsbewegung der rektalen Muskulatur, wird die resultierende Kraft vom intrarektalen Ballonabschnitt 3 aufgenommen. Die im gesamten Okklusionselement 1 resultierende Drucksteigerung führt zu einer aktiven Aufrichtung des transanalen Abschnitts 11 und 5, wobei sich das abführende transanale Lumen aufweitet und sich, bei entsprechend großer Steigerung des Ballonfülldrucks, in seiner gesamten Querschnittsfläche darstellt. Ein weiterer Vorteil dieser Druck- und Volumenbewegung vom intrarektalen in den transanalen Abschnitt besteht in der aktiven Entwindung bzw. Detorquierung des transanalen Segmentes. Ohne einen derartigen Entwindungsmechanismus kann ein funktionsbeeinträchtigender Lumenverschluß infolge einer axialen Verdrehung im transanalen Segment der Vorrichtung nicht sicher ausgeschlossen werden. Die den spontanen Defäkationsvorgang aktiv unterstützende Öffnung des Analkanals kann auch gezielt vom Anwender eingestellt werden. Soll zum Beispiel eine Spülung des Darms (Klistier) durchgeführt werden, kann der Fülldruck in der Vorrichtung 1 für die Zeitdauer der Spülung entsprechend angehoben werden. Es wirken dann mehrere Funktionskomponenten günstig zusammen. Die axial gerichtete Bewegung der intrarektalen Ballonkomponente schmiegt den intrarektalen Ballonkörper 3 dichtend an den zu verschließenden Anus, wobei der präanale Widerlagerballon 4 mit gleicher Kraft von außen an den Anus herangezogen wird, zudem richtet sich das drainierende Lumen des transanalen Segmentes 5 und 11 auf, eventuelle Verwindungen über die Längsachse der Vorrichtung entwinden sich.

Im Bereich der patientenproximalen Erweiterung 3 wird das innere Lumen 13 von einer Versteifungshülse 14 offengehalten, deren Länge vorzugsweise gleich oder kleiner ist als die axiale Erstreckung der radialen, intrarektalen Erweiterung 3 des Ballons 2. Die Materialhärte der Versteifungshülse 14 ist vorzugsweise gleich oder kleiner als die Materialhärte des Ballons; ihre Steifigkeit erhält die Hülse 14 aus ihrer erhöhten Wandstärke. Bevorzugt kann für die Hülse 14 das selbe Material verwendet werden wie für den Schlauch bzw. Ballon 2. Dadurch erleichtert sich die Fixierung der Hülse 14 innerhalb des inneren Lumens 13, Insbesondere durch Verklebung mit der Innenlage 6 des Ballons 2, wobei solchenfalls ein das betreffende Material anlösendes Mittel als Klebstoff bzw. zur Verschweißung verwendet werden kann.

Vorzugsweise innerhalb des inneren Lumens 13 erstreckt sich ein Schlauch 15 von dem patientenproximalen Ende 3 bis jenseits des Konnektorstücks 9. Durch diesen Schlauch 15, der an dem Ballon 2 - vorzugsweise an dessen Innenlage 6 - fixiert sein kann, bspw. durch Verkleben, läßt sich ein Spülmedium in den Darm des Patienten einleiten. Damit solchenfalls keine Bakterien aus dem transanalen Bereich 11 oder aus dem Bereich jenseits der transanalen Erweiterung 4 in den Darm geschwemmt werden können, befindet sich die Öffnung des Schlauchs 15 an dem vordersten Bereich des intrarektalen Endes 3 der Vorrichtung 1. Der Schlauch 15 kann innen durch die Versteifungshülse 14 geführt sein oder zwischen derselben und der Innenlage 6 hindurch.

Um während eines Einlaufs das zentrale Lumen 13 optimal versperren zu können, ist weiterhin ein Okklusionsballon 16 vorgesehen. Dieser sitzt bevorzugt innerhalb der Versteifungshülse 14 und hat bei der Ausführungsform nach den Fig. 1 und 2 eine kugelförmige Präformierung mit einem Durchmesser, der etwas größer ist als der Durchmesser der Versteifungshülse 14. Dadurch verschließt der inflatierte Okklusionsballon 16 das zentrale Lumen 13 vollständig und liegt unter Druck sowie unter geringer Verformung rundum an der Innenseite der Versteifungshülse 14 an, wodurch dieselbe verschlossen wird.

An dem Okklusionsballon 16 mündet ein weiterer Schlauch 17, mit dem ein vorzugsweise gasförmiges Druckmedium in den Okklusionsballon 16 geleitet werden kann, um das Lumen 13 im Bereich der Versteifungshülse 14 zu verschließen. Der Schlauch 17 ist an der Innenseite der Ballon-Innenlage 6 bis durch das Konnektorstück 9 hindurch geführt und somit von außen zugänglich. Etwa im Bereich der Mündung des Schlauchs 17 in den Okklusionsballon 16 ist letzterer an der Versteifungshülse 14 fixiert, bspw. etwa punktuell angeklebt.

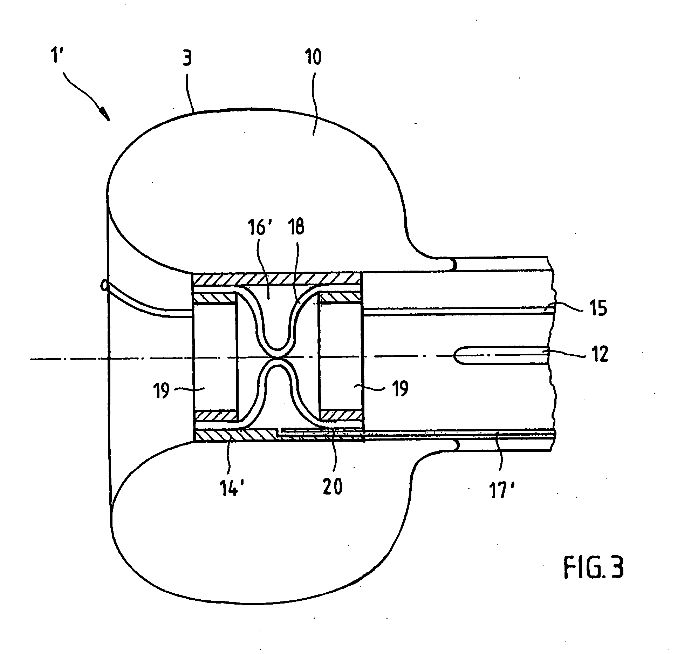

Die Ausführungsform 1' aus Fig. 3 unterscheidet sich von der vorangehenden nur durch eine andere Gestalt des Okklusionsballons 16'. Dieser hat bei letzterer Ausführungsform 1' die Gestalt eines kurzen Schlauchs 18, etwa von dem Durchmesser der Versteifungshülse 14 und auch etwa von deren Länge. Dieser Schlauch 18 kann in seinem axialen Mittelabschnitt in Form einer radialen Erweiterung präformiert sein. Die beiden Enden dieses Schlauchs 18 werden an der Innenseite der Versteifungshülse 14 fixiert, bspw. durch Einklemmen zwischen derselben und je einer eingesteckten Innenhülse 19. Die beiden Innenhülsen 19 können zusätzlich mit Klebstoff in der Versteifungshülse 14 festgelegt sein und bilden sodann mit dieser eine bautechnische Einheit. Wird der Hohlraum 16' zwischen dem mittleren, nicht festgeklemmten Bereich des Schlauchs 18 und der Versteifungshülse 14 von außen her mit einem Fluid gefüllt, so wölbt sich der Schlauch 18 dort nach innen, wie dies in Fig. 3 zu sehen ist, und verschließt das Lumen 13 nach Art einer Iris. Zum Einfüllen eines Fluids ist in diesem Fall die Versteifungshülse 14 mit einem Kanal 20 versehen, der diese von der patientendistalen Stirnseite her bis zu dem Bereich zwischen den beiden Innenhülsen 19 durchsetzt und dort nach innen mündet. Der Schlauch 17' kommuniziert mit dem Kanal 20.

## Patentansprüche

1. Vorrichtung (1;1') zum Verschließen eines natürlichen oder künstlichen Darmausgangs, umfassend einen aufblasbaren Ballon (2) mit einer etwa torusförmigen Struktur, gebildet aus einem flächigen, in sich umgestülpten Schlauchabschnitt, dessen beide Enden (7,8) etwa koaxial ineinander verlaufen und mit einer Hülse (9) verbunden sind, wobei eine äußere Lage (5) des umgestülpten Schlauchabschnittes einen radial erweiterten, intrarektalen, patientenproximalen Bereich (3) zum Einführen in das Rektum und einen demgegenüber verjüngten, transanalen, patientendistalen Bereich (11) aufweist, der während des Gebrauchs zumindest bereichsweise außerhalb des Rektums verbleibt, und wobei die Schlauchabschnitte in dem transanalen Bereich (11) eine nach Shore-Härteprüfung A bestimmbare Materialhärte H₁ aufweisen, die größer ist als 60, **dadurch gekennzeichnet, dass** innerhalb des intrarektalen Bereichs (3) einer inneren Lage (6) des umgestülpten Schlauchabschnitts eine Versteifungshülse (14) derart eingesetzt ist, dass diese von einem Hohlraum (10) innerhalb des Ballons (2) durch dessen innere, durchgängige Lage (6) vollständig getrennt ist, wobei die Versteifungshülse (14) mit keinem der Enden (7,8) des den Ballon (2) bildenden Schlauchabschnitts verbunden ist.

2. Vorrichtung (1;1') nach Anspruch 1, **dadurch gekennzeichnet, dass** die nach Shore-Härteprüfung A bestimmbare Materialhärte H₁ der inneren Lage (6) und der äußeren Lage (5) der Schlauchabschnitte in dem transanalen Bereich (11) größer ist als 70, vorzugsweise größer als 80.

3. Vorrichtung (1;1') nach Anspruch 1 oder 2, **dadurch gekennzeichnet, dass** die Wandstärke des Ballons (2), vorzugsweise in seinem intrarektalen Bereich (3), insbesondere im Bereich seiner äußeren Lage (5), kleiner ist als 50 µm, vorzugsweise kleiner als 40 µm, insbesondere kleiner als 30 µm.

4. Vorrichtung (1;1') nach einem der Ansprüche 1 bis 3, **dadurch gekennzeichnet, dass** der den Ballon (2) bildende Schlauchabschnitt aus Polyurethan besteht.

5. Vorrichtung (1;1') nach einem der vorhergehenden Ansprüche, **dadurch gekennzeichnet, dass** die Versteifungshülse (14) eine nach Shore-Härteprüfung A bestimmbare Materialhärte H₂ aufweist, die gleich oder kleiner ist als die nach Shore-Härteprüfung A bestimmbare Materialhärte H₁ der inneren Lage (6) und der äußeren Lage (5) der Schlauchabschnitte in dem transanalen Bereich (11).

6. Vorrichtung (1;1') nach einem der vorhergehenden Ansprüche, **dadurch gekennzeichnet, dass** der Ballon (2) und die Versteifungshülse (14) aus dem selben Material gefertigt sind.

7. Vorrichtung (1;1') nach einem der vorhergehenden Ansprüche, **dadurch gekennzeichnet, dass** die Härte und Wandstärke der Versteifungshülse (14) derart gewählt sind, dass diese zum Einführen in das Rektum radial zusammengedrückt werden kann.

8. Vorrichtung (1;1') nach einem der vorhergehenden Ansprüche, **dadurch gekennzeichnet, dass** ein oder vorzugsweise beide Enden (7,8) des den Ballon bildenden Schlauchabschnitts in dem oder jenseits, d.h. patientenfern des transanalen Bereichs (11) liegen.

9. Vorrichtung (1;1') nach einem der vorhergehenden Ansprüche, **dadurch gekennzeichnet, dass** eine wenigstens mit einem Ende (7,8) des den Ballon (2) bildenden Schlauchabschnitts verbundene Hülse (9) als extrakorporales Konnektorstück ausgebildet ist.

10. Vorrichtung (1;1') nach einem der vorhergehenden Ansprüche, **dadurch gekennzeichnet, dass** der ein abgeschlossenes Volumen bildende Hohlraum (10) innerhalb des Ballons (2) von außen mit Druck beaufschlagbar ist.

11. Vorrichtung (1;1') nach Anspruch 11, **dadurch gekennzeichnet, dass** ein von dem Ballon (2) umgebener Durchgang (13) zur Entleerung des Darms vorgesehen ist, indem der ein abgeschlossenes Volumen bildende Hohlraum (10) durch zwei etwa konzentrisch zueinander liegende, innere und äußere Lagen (5,6) des Ballons (2) begrenzt wird.

12. Vorrichtung (1;1') nach Anspruch 11, **dadurch gekennzeichnet, dass** zur Offenhaltung des Durchgangs (13) zur Entleerung des Darms in demjenigen Bereich der Vorrichtung (1;1'), die in dem Analkanal liegt, die innere Lage (6) des Ballons (2) punkt-, linien- oder flächenmäßig mit der äußeren Lage (5) des Ballons (2) verbunden ist. bspw. durch Verschweißungen (12) oder Verklebungen.

13. Vorrichtung (1;1') nach einem der vorhergehenden Ansprüche, **gekennzeichnet durch** einen Okklusionsballon (16;16') zum Verschließen des Durchgangs (13) zur Entleerung des Darms.

14. Vorrichtung (1;1') nach Anspruch 13, **dadurch gekennzeichnet, dass** der Okklusionsballon (16;16') innerhalb der Versteifungshülse (14) positioniert ist.

15. Vorrichtung (1;1') nach Anspruch 13 oder 14, **dadurch gekennzeichnet, dass** der Okklusionsballon (16; 16') aus einem dünnwandigen, entsprechend präformierten Material gebildet ist.

16. Vorrichtung (1;1') nach Anspruch 13 oder 14, **dadurch gekennzeichnet, dass** der Okklusionsballon (16;16') aus einem völlig elastisch zurückstellbaren, zum Ballon ausformbaren Kompartiment gebildet ist.

17. Vorrichtung (1;1') nach einem der vorhergehenden Ansprüche, **dadurch gekennzeichnet, dass** im Bereich der Versteifungshülse (14) eine Spülöffnung vorgesehen ist, die mit einem entlang des transanalen Abschnitts verlaufenden Kanal (15) zum Einleiten eines Spülfluids verbunden ist.

18. Vorrichtung (1;1') nach einem der vorhergehenden Ansprüche, **dadurch gekennzeichnet, dass** sich der Kanal (15) zum Einleiten eines Spülfluids bis zu dem intrarektalen Bereich (3) erstreckt.

19. Vorrichtung (1;1') nach einem der vorhergehenden Ansprüche, **gekennzeichnet durch** eine radiale Erweiterung (4) in dem transanalen Bereich (11).

20. Vorrichtung (1;1') nach Anspruch 19, **dadurch gekennzeichnet, dass** die radiale Erweiterung (4) des die äußere Lage (5) des den Ballon (2) bildenden Schlauchabschnitts eine Präformierung mit einer nach außen weisenden Erweiterung aufweist.

21. Vorrichtung (1;1') nach Anspruch 20, **dadurch gekennzeichnet, dass** die eine Präformierung bildende, nach außen weisende Erweiterung (4) in dem transanalen Bereich (11) der äußeren Lage (5) des Ballons (2) eine ring- oder scheibenförmige Geometrie aufweist.

22. Vorrichtung (1;1') nach Anspruch 20, **dadurch gekennzeichnet, dass** die eine Präformierung bildende, nach außen weisende Erweiterung (4) in dem transanalen Bereich (11) der äußeren Lage (5) des Ballons (2) die Geometrie eines oder zweier, etwa diametral voneinander weg divergierender Finger aufweist.

23. Vorrichtung (1;1') nach Anspruch 20, **dadurch gekennzeichnet, dass** über der äußeren Lage (5) des den Ballon (2) bildenden Schlauchabschnitts in dem transanalen Bereich (11) ein keilförmiges Element vorgesehen, insbesondere befestigt ist.

## Claims

1. Device (1;1') for sealing a natural or artificial intestinal outlet, comprising an inflatable balloon (2) with a roughly toroidal structure, formed of a flat, everted balloon segment whose two ends (7,8) extend roughly coaxially one inside the other and are each connected to a (respective) sleeve (9), the outer layer (5) of the everted tube segment comprising a radially expanded, intrarectal, patient-proximal region (3) that is to be inserted in the rectum and a transanal, patient-distal region (11) narrowed with respect thereto, that remains at least regionally outside the rectum during use, and said tube segments having in the transanal region (11) a material hardness H₁, determinable according to the Shore A hardness test, that is greater than 60, **characterized in that** a stiffening sleeve (14) is inserted in the intrarectal region (3) of said inner layer (6) of the everted tube segment in such fashion as to be completely separated from a hollow space (10) inside the balloon (2) by the continuous inner layer (6) thereof, whereby said stiffening sleeve (14) is not connected to either end (7,8) of the tube segment forming said balloon (2).

2. Device (1;1') according to claim 1, **characterized in that** the material hardness H₁, determinable according to the Shore A hardness test, of said inner layer (6) and said outer layer (5) of said tube segments in said transanal region (11) is greater than 70, preferably greater than 80.

3. Device (1;1') according to claim 1 or 2, **characterized in that** the wall thickness of said balloon (2), preferably in its intrarectal region (3), particularly in the region of its outer layer (5), is less than 50 µm, preferably less than 40 µm, particularly less than 30 µm.

4. Device (1;1') according to one of claims 1 to 3, **characterized in that** the tube segment forming said balloon (2) is made of polyurethane.

5. Device (1;1') according to one of the preceding claims, **characterized in that** said stiffening sleeve (14) has a material hardness H₂, determinable according to the Shore A hardness test, that is equal to or less than the material hardness H₁, determinable according to the Shore A hardness test, of said inner layer (6) and said outer layer (5) of the tube segments in said transanal region (11).

6. Device (1;1') according to one of the preceding claims, **characterized in that** said tube (2) and said stiffening sleeve (14) are made of the same material.

7. Device (1;1') according to one of the preceding claims, **characterized in that** the hardness and wall thickness of said stiffening sleeve (14) are selected such that the aforesaid elements can be compressed radially for insertion in the rectum.

8. Device (1;1') according to one of the preceding claims, **characterized in that** one or preferably both ends (7,8) of the tube segment forming said balloon (2) are disposed in said transanal region (11) or therebeyond, that means distal with regard to the patient.

9. Device (1;1') according to one of the preceding claims, **characterized in that** a sleeve (9) connected to at least one end (7,8) of the tube segment forming said balloon (2) is configured as an extracorporeal connector element.

10. Device (1;1') according to one of the preceding claims, **characterized in that** a hollow space (10) inside said balloon (2) forming a sealed-off volume can be pressurized from the outside.

11. Device (1;1') according to claim 10, **characterized in that** a passage (13) surrounded by said balloon (2) is provided to empty the bowel **in that** the hollow space (10) forming a sealed-off volume is bounded by two roughly mutually concentric inner and outer layers (5, 6) of said balloon (2).

12. Device (1;1') according to claim 11, **characterized in that** to keep the bowel emptying passage (13) open in the region of said device (1;1') residing in the anal canal, the inner layer (6) of said balloon (2) is connected punctiformly, linearly or areally to the outer layer (5) of said balloon (2), for example by welds (12) or adhesive bonds.

13. Device (1;1') according to one of the preceding claims, **characterized by** an occluding balloon (16;16') for sealing said bowel emptying passage (13).

14. Device (1;1') according to claim 13, **characterized in that** said occluding balloon (16;16') is positioned inside said stiffening sleeve (14).

15. Device (1;1') according to claim 13 or 14, **characterized in that** said occluding balloon (16;16') is formed from a thin-walled, suitably preshaped material.

16. Device (1;1') according to claim 13 or 14, **characterized in that** said occluding balloon (16;16') is formed from an entirely elastically restorable compartment that can be shaped into said balloon.

17. Device (1;1') according to one of the preceding claims, **characterized in that** provided in the region of said stiffening sleeve (14) is a flushing opening connected to a conduit (15) that extends along the transanal segment and serves to introduce a flushing fluid.

18. Device (1;1') according to one of the preceding claims, **characterized in that** said conduit (15) for introducing a flushing fluid extends to the intrarectal segment (3).

19. Device (1;1') according to one of the preceding claims, **characterized by** a radial expansion (4) in said transanal region (11).

20. Device (1;1') according to claim 19, **characterized in that** the radial expansion (4) of the tube segment forming the outer layer (5) of the said balloon (2) is a preshape (4) comprising an outwardly oriented expansion.

21. Device (1;1') according to claim 20, **characterized in that** the outwardly oriented expansion (4) forming a preshape in said transanal region (11) of said outer layer (5) of said balloon (2) has a ring- or disk-shaped geometry.

22. Device (1;1') according to claim 20, **characterized in that** the outwardly oriented expansion (4) forming a preshape in said transanal region (11) of said outer layer (5) of said balloon (2) comprises the geometry of one or two fingers that diverge diametrically from each other.

23. Device (1;1') according to claim 20, **characterized in that** a wedge-shaped element is provided, particularly fastened, in said transanal region (11) over said outer layer (5) of said tube segment forming said balloon (2).

## Revendications

1. Dispositif (1 ; 1') permettant de fermer un anus naturel ou artificiel, comprenant un ballonnet (2) gonflable de structure approximativement toroïdale, formé à partir d'une section de tuyau flexible plate, retournée sur elle-même, dont les deux extrémités (7, 8) s'étendent l'une dans l'autre de manière approximativement coaxiale et qui sont reliées à un manchon (9) ; une couche extérieure (5) de la section de tuyau flexible retournée présentant une zone (3) élargie radialement, intrarectale, proximale du patient, à introduire dans le rectum et une zone (11) en comparaison rétrécie, transanale, distale du patient, qui demeure au moins en partie en dehors du rectum durant l'utilisation ; les sections de tuyau flexible présentant dans la zone transanale (11) une dureté de matériau H₁ pouvant être déterminée par essai de dureté Shore A qui est supérieure à 60, **caractérisé en ce qu'**un manchon de renfort (14) est inséré dans la zone intrarectale (3) d'une couche intérieure (6) de la section de tuyau flexible retournée de sorte que ledit manchon de renfort soit entièrement séparé d'une cavité (10) à l'intérieur du ballonnet (2) par sa couche intérieure (6) pouvant être traversée ; le manchon de renfort (14) n'étant relié à aucune des extrémités (7, 8) de la section du tuyau flexible formant le ballonnet (2).

2. Dispositif (1 ; 1') selon la revendication 1, **caractérisé en ce que** la dureté de matériau H₁ pouvant être déterminée par essai de dureté Shore A de la couche intérieure (6) et de la couche extérieure (5) des sections de tuyau flexible dans la zone transanale (11) est supérieure à 70, de préférence supérieure à 80.

3. Dispositif (1 ; 1') selon la revendication 1 ou 2, **caractérisé en ce que** l'épaisseur de paroi du ballonnet (2), de préférence dans sa zone intrarectale (3), en particulier dans la zone de sa couche extérieure (5), est inférieure à 50 µm, de préférence inférieure à 40 µm, en particulier inférieure à 30 µm.

4. Dispositif (1 ; 1') selon l'une des revendications 1 à 3, **caractérisé en ce que** la section de tuyau flexible formant le ballonnet (2) est en polyuréthane.

5. Dispositif (1 ; 1') selon l'une des revendications précédentes, **caractérisé en ce que** le manchon de renfort (14) présente une dureté de matériau H₂ pouvant être déterminée par essai de dureté Shore A qui est égale ou inférieure à la dureté de matériau H₁ pouvant être déterminée par essai de dureté Shore A de la couche intérieure (6) et de la couche extérieure (5) des sections de tuyau flexible dans la zone transanale (11).

6. Dispositif (1 ; 1') selon l'une des revendications précédentes, **caractérisé en ce que** le ballonnet (2) et le manchon de renfort (14) sont réalisés en un même matériau.

7. Dispositif (1 ; 1') selon l'une des revendications précédentes, **caractérisé en ce que** la dureté et l'épaisseur de la paroi du manchon de renfort (14) sont choisies de sorte que ce dernier puisse être ccmprimé radialement pour l'introduire dans le rectum.

8. Dispositif (1 ; 1') selon l'une des revendications précédentes, **caractérisé en ce que** l'une ou de préférence les deux extrémités (7, 8) de la section de tuyau flexible formant le ballonnet sont dans ou au-delà, c.-à-d. éloigné du patient, de la zone transanale (11).

9. Dispositif (1 ; 1') selon l'une des revendications précédentes, **caractérisé en ce qu'**un manchon (9) relié au moins à une extrémité (7, 8) de la section de tuyau flexible formant le ballon (2) est réalisé sous forme d'élément connecteur extracorporel.

10. Dispositif (1 ; 1') selon l'une des revendications précédentes, **caractérisé en ce que** la cavité (10) qui forme un volume fermé à l'intérieur du ballonnet (2) est exposé de l'extérieur à une pression.

11. Dispositif (1 ; 1') selon la revendication 11, **caractérisé en ce qu'**un passage (13) entouré par le ballonnet (2) est prévu pour vider l'intestin en délimitant la cavité (10) qui forme un volume fermé par deux couches intérieures et extérieures (5, 6), approximativement concentriques l'une par rapport à l'autre, du ballonnet (2).

12. Dispositif (1 ; 1') selon la revendication 11, **caractérisé en ce que**, pour maintenir ouvert le passage (13) pour vider l'intestin dans la zone du dispositif (1 ; 1') qui est dans le canal anal, la couche intérieure (6) du ballonnet (2) est reliée ponctuellement, linéairement ou superficiellement à la couche extérieure (5) du ballonnet (2), par exemple par des soudures (12) ou des collages.

13. Dispositif (1 ; 1') selon l'une des revendications précédentes, **caractérisé par** un ballon d'occlusion (16 ; 16') destiné à la fermeture du passage (13) pour vider l'intestin.

14. Dispositif (1 ; 1') selon la revendication 13, **caractérisé en ce que** le ballon d'occlusion (16 ; 16') est positionné à l'intérieur du manchon de renfort (14).

15. Dispositif (1 ; 1') selon la revendication 13 ou 14, **caractérisé en ce que** le ballon d'occlusion (16 ; 16') est réalisé en un matériau à paroi mince, préformé en conséquence.

16. Dispositif (1 ; 1') selon la revendication 13 ou 14, **caractérisé en ce que** le ballon d'occlusion (16 ; 16') est réalisé à partir d'un compartiment reprenant entièrement élastiquement sa forme initiale, pouvant être formé en ballonnet.

17. Dispositif (1 ; 1') selon l'une des revendications précédentes, **caractérisé en ce que** dans la zone du manchon de renfort (14) est prévu un orifice de rinçage qui est relié à un canal (15) s'étendant à la section transanale pour introduire un fluide de rinçage.

18. Dispositif (1 ; 1') selon l'une des revendications précédentes, **caractérisé en ce que** le canal (15) pour introduire un fluide de rinçage s'étend jusqu'à la zone intrarectale (3).

19. Dispositif (1 ; 1') selon l'une des revendications précédentes, caractérisé en un élargissement radial (4) dans la zone transanale (11).

20. Dispositif (1 ; 1') selon la revendication 19, **caractérisé en ce que** l'élargissement radial (4) de la section de tuyau flexible formant la couche extérieure (5) du ballonnet (2) est un préformage avec un élargissement montrant vers l'extérieur.

21. Dispositif (1 ; 1') selon la revendication 20, **caractérisé en ce que** l'élargissement (4) montrant vers l'extérieur, formant un préformage dans la zone transanale (11) de la couche extérieure (5) du ballonnet (2) présente une géométrie annulaire ou en forme de disque.

22. Dispositif (1 ; 1') selon la revendication 20, **caractérisé en ce que** l'élargissement (4) montrant vers l'extérieur, formant un préformage dans la zone transanale (11) de la couche extérieure (5) du ballonnet (2) présente la géométrie d'un ou de deux doigts divergents approximativement diamétralement l'un de l'autre.

23. Dispositif (1 ; 1') selon la revendication 20, **caractérisé en ce que**, au-dessus de la couche extérieure (5) de la section de tuyau flexible formant le ballon (2) dans la zone transanale (11) est prévu un élément cunéiforme, en particulier qui y est fixé.
